# EUROPEAN PATENT SPECIFICATION

(11) **EP 2 975 410 B1**
(45) Date of publication and mention of the grant of the patent: **31.01.2018**
(21) Application number: 14764892.7
(22) Date of filing: 12.03.2014
(51) Int. Cl.: G01N 33/68, A61K 8/64, A61Q 19/02, C12Q 1/68, G01N 33/50

(54) **METHOD FOR SCREENING SUBSTANCE HAVING WHITENING EFFICACY**
VERFAHREN ZUM SCREENING NACH EINER SUBSTANZ MIT AUFHELLENDER WIRKUNG
PROCÉDÉ DE CRIBLAGE DE SUBSTANCE AYANT UNE EFFICACITÉ DE BLANCHISSEMENT

(30) Priority: 13.03.2013 KR 20130026604
(43) Date of publication of application: 20.01.2016
(73) Proprietor: Amorepacific Corporation, Seoul 140-777 (KR)
(72) Inventor: KIM, Jue Won, Yongin-si Gyeonggi-do 446-729 (KR); CHOI, Hyun Jung, Yongin-si Gyeonggi-do 446-729 (KR); LEE, Tae Ryong, Yongin-si Gyeonggi-do 446-729 (KR); CHO, Eun Gyung, Yongin-si Gyeonggi-do 446-729 (KR)
(74) Representative: Brevalex
(86) International application number: PCT/KR2014/002066
(87) International publication number: WO 2014/142552

(56) References cited:
- WO-A2-2012/057567
- JP-A- 2010 081 913
- KR-A- 20090 054 505
- KR-A- 20100 039 077
- KR-A- 20100 138 918
- KR-A- 20110 057 147
- J. M. GILLBRO ET AL: "The melanogenesis and mechanisms of skin-lightening agents - existing and new approaches", INTERNATIONAL JOURNAL OF COSMETIC SCIENCE, 1 January 2011 (2011-01-01), pages no-no, XP055000016, ISSN: 0142-5463, DOI: 10.1111/j.1468-2494.2010.00616.x
- GENJI IMOKAWA ET AL: "Inhibitors of Intracellular Signaling Pathways that Lead to Stimulated Epidermal Pigmentation: Perspective of Anti-Pigmenting Agents", INTERNATIONAL JOURNAL OF MOLECULAR SCIENCES 2012 MDPI AG CHE, vol. 15, no. 5, 12 May 2014 (2014-05-12), pages 8293-8315, XP055287377, ISSN: 1661-6596, DOI: 10.3390/ijms15058293
- THANIGAIMALAI PILLAIYAR ET AL: "Inhibitors of melanogenesis: a patent review (2009 - 2014)", EXPERT OPINION ON THERAPEUTIC PATENTS., vol. 25, no. 7, 4 May 2015 (2015-05-04), pages 775-788, XP055288722, GB ISSN: 1354-3776, DOI: 10.1517/13543776.2015.1039985
- KIM ET AL.: 'FoxO3a Is an Antimelanogenic Factor that Mediates Antioxidant-Induced Depigmentation' JOURNAL OF INVESTIGATIVE DERMATOLOGY vol. 134, no. 5, 02 January 2014, pages 1378 - 1388, XP055282679 DOI: 10.1038/JID.2013.510

## Description

### TECHNICAL FIELD

The present invention relates, in general, to a method for screening a substance having a whitening effect, and more particularly, to a method of determining whether or not a test substance has a whitening effect by analyzing whether or not the test substance activates the longevity gene Fox03 (Forkhead box 03) or stimulates the expression of the Fox03 gene, by use of the Fox03a (Forkhead box 03A) protein that is encoded by the Fox03 gene.

### BACKGROUND ART

Melanin is a pigment that determines the color of the human skin, and the skin color is determined by the amount and distribution of melanin. Melanin is produced in cells called melanocytes in the epidermis, which are detached after migration to the skin surface by skin metabolism. The number of melanocytes is nearly the same regardless of skin color. In other words, skin color varies from person to person, because the amount, type and distribution of melanin produced vary from person to person. In the skin, tyrosine is converted into DOPA by the enzyme tyrosinase, and melanin that is a dark brown polymer is finally produced by a series of oxidations of DOPA.

Melanin is not degraded *in vivo,* and is removed from the skin when keratinocytes are detached from the epidermis together with melanin. If melanin is excessively produced, it will cause hyperpigmentations, such as discoloration, freckles and spots, which are unfavorable in terms of beauty. Thus, the need to prevent melanin pigmentation caused by UV light in outdoor activities such as exercise has increased. Accordingly, there has been an increasing demand for the development of whitening agents for preventing the excessive production of melanin.

It has become an important issue to find a substance that exhibits a whitening effect by inhibiting the production of melanin, and there has been a need for a method capable capable of analyzing and determining the whitening effect of a test substance by subjective criteria without using sensory evaluation or visual evaluation.

Examples of methods for screening a substance having a whitening effect can be found in documents **[1], [2], [3], [4],** [6] and [7]. It was known from [5] that Fox03a is involved in ageing.

### DISCLOSURE

### Technical Problem

Accordingly, the present invention have found that whether or not a test substance has a whitening effect can be determined by use of the Fox03a (Forkhead box 03A) protein that is encoded by the longevity gene Fox03 (Forkhead box 03), thereby completing the present invention.

Therefore, it is an object of the present invention to provide a method of screening a substance having a whitening effect by use of Fox03a (Forkhead box 03A).

### Technical Solution

In order to accomplish the above object, the present invention provides a method for screening a substance having a whitening effect, the method comprising analyzing whether or not a test substance activates Fox03a (Forkhead box 03A) or stimulates the expression of Fox03a.

Fox03a (Forkhead box 03A) according to the present invention is a protein that is encoded by Fox03 known as a longevity gene. It is a transcription factor that is involved in the insulin/IGF-like signaling pathway (IIS) and acts on the expression of enzymes such as Mn-SOD and catalase. If Fox03a is activated, it will exhibit an anti-aging effect by activating *in vivo* defense mechanisms. The activation of Fox03a can be determined by the migration of Fox03a to the nucleus. In other words, Fox03 migrates from the cytoplasm to the nucleus to initiate the expression of enzymes.

The present inventors have found that the extent of migration of Fox03a to the nucleus by the activation of Fox03a is proportional to the decrease in melanin production. Thus, whether a candidate substance expected to have a whitening effect exhibits the whitening effect by reducing melanin production can be simply and subjectively analyzed by treating skin cells with the candidate substance and analyzing whether or not the candidate substance activates Fox03a or stimulates the expression of Fox03a.

Accordingly, the present invention provides a method for screening a substance having a whitening effect, the method comprising analyzing whether or not a test substance activates the gene encoding the Fox03a (Forkhead box 03A) protein or stimulates the expression of the gene.

In an embodiment of the present invention, analysis of whether or not the test substance activates Fox03a may comprise the steps of: a) treating skin cells with the test substance; and b) observing migration of Fox03a in the skin cells treated with the test substance in step a).

The screening method according to the present invention may further comprise step c) of determining that the test substance is the substance having a whitening effect, if the Fox03a protein migrates to the nucleus in the skin cells. This is because it can be considered that the Fox03a protein-encoding gene is activated by the migration of Fox03a to the nucleus. In addition, because the substance determined to have a whitening effect by the screening method can initiate the expression of antioxidant enzymes by the activation of Fox03a, it can be determined to be a substance that exhibits a whitening effect together with anti-aging and antioxidant effects.

In step a), the test substance is preferably used at a concentration ranging from 1 nM to 100 nM depending on the kind of test substance. If the test substance is used at a concentration of less than 1 nM, the effective concentration of the test substance will not be reached, and if the test substance is used at a concentration of 100 nM, it can cause abnormality in cell homeostasis due to the excessive effect thereof, and thus the migration of Fox03a to the nucleus and the whitening effect of the test substance will decrease rather than increase.

The skin cells may be keratinocytes, fibroblasts or melanocytes. In an embodiment of the present invention, analysis of whether the test substance stimulates the expression of Fox03a can be performed using Western blotting, ELISA or RT-PCR.

If the test substance is determined to stimulate the expression of Fox03a, it can be determined to be a substance having a whitening effect. In addition, because the substance determined to have a whitening effect by the screening method can initiate the expression of antioxidant enzymes due to the characteristics of the longevity gene Fox03, it can be determined to be a substance exhibiting not only the whitening effect found in the present invention, but also anti-aging and antioxidant effects.

### Advantageous Effects

The method for screening a substance having a whitening effect according to the present invention can determine whether or not a test substance has a whitening effect, by subjective criteria, because a decrease in melanin formation is proportional to the extent of activation of Fox03a. Thus, according to the screening method of the present invention, a substance having a whitening effect can be screened in a rapid, simple and accurate manner without using a method such as a clinical test or sensory evaluation. In addition, a substance determined to have a whitening effect by the screening method of the present invention can initiate the expression of antioxidant enzymes by Fox03a, and thus a substance exhibiting a whitening effect together with anti-aging and antioxidant effects can be screened.

### DESCRIPTION OF DRAWINGS

FIG. 1a is a photograph showing a comparison of the color of a cell pellet lysate between MNT-1 cells (NT), MNT-1 cells transfected with a wild-type Fox03a plasmid (WT), and MNT-1 cells transfected with an NLS mutant Fox03a plasmid having a mutation in the nuclear location sequence (NLS).
FIG. 1b is a photograph showing the results of Western blot analysis of MNT-1 cells (NT), MNT-1 cells transfected with a wild-type Fox03a plasmid (WT), and MNT-1 cells transfected with an NLS mutant Fox03a plasmid having a mutation in the nuclear location sequence (NLS).
FIG. 2 depicts graphs showing the melanin amount and tyrosinase activity of MNT-1 cells treated with each of vitamin C, N-acetylcysteine and Trolox.
FIG. 3 depicts fluorescence microscope images showing the observation of the migration of Fox03a to the nuclei of MNT-1 cells treated with each of vitamin C, N-acetylcysteine and Trolox.

### BEST MODE

Hereinafter, the present invention will be described in further detail with reference to examples and test examples. These examples are for illustrative purposes only and are not intended to limit the scope of the present invention, and those skilled in the art will appreciate that various modifications, substitutions and additions may be made to these examples and also fall within the scope of the present invention.

### Test Example 1: Experiment on Activation of FoxO3a and Decrease in Melanin Production

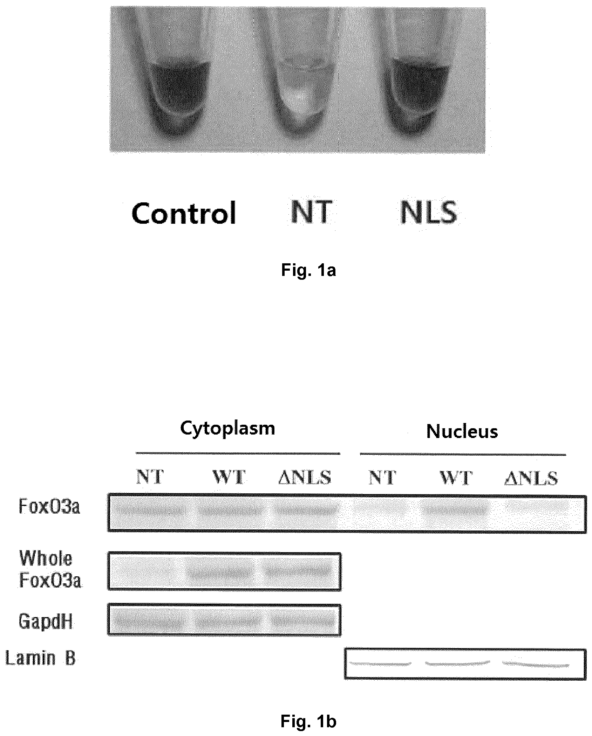

1×10⁶ MNT-1 cells (human melanoma cell line, Lonza, SWISS) were added to MEM medium (10% DMEM) and transfected with each of a wild-type (WT) Fox03a plasmid (Plasmid 1787: HA-FOX03a WT, Addgene) and an NLS (nuclear localization sequence) mutant Fox03a plasmid (Plasmid 8361: FLAG-FOXO3a TM, Addegene) using Lipofectamin 2000 (Invitrogen), followed by culture at 37°C for 48 hours. The MNT-1 cells were lysed with 500 µL of LIPA buffer (25 mM Tris-HCl, pH 7.4, 0.1% SDS, 0.1% Triton X-100, 1% sodium deoxycholate, 150 mM NaCl, 1 mM, EDTA, 1 mM Na₃VO₄,1 mM PMSF, 10 mg/mL aprotinin, 5 mg/mL leupeptin), and the cell lysates were centrifuged (Centrifuge 5415R, Eppendorf, Germany). The resulting pellets were separated, and the color thereof was observed. FIG. 1a is a photograph showing the colors of lysates obtained by lysing the contents of the pellets with 200 µL of 1N sodium hydroxide. As a control, MNT-1 cells transfected with no plasmid were used.

20 µg of each of the lysates was loaded, and the expression level of the FoxO3a protein in the cells was analyzed by Western blotting using Gapdh as a marker protein for the cytoplasm and Lamin B as a marker protein for the nucleus. The results of the analysis are shown in FIG. 1b.

From the results in FIG. 1a, it can be seen that, when the MNT-1 cells were transfected with the wild-type Fox03a plasmid, melanin production in the cells was significantly reduced by the overexpression of Foxo3a, and when the MNT-1 cells were transfected with the NLS mutant Fox03a plasmid having a mutation in the nuclear location sequence, no decrease in melanin production appeared, because Fox03a did not migrate to the nucleus. In addition, from the results in FIG. 1b, it can be seen that, when the MNT-1 cells were transfected with the wild-type Fox03a plasmid, Fox03a was activated and migrated to the nucleus. Thus, it can be seen that, if FoxO3a is not activated, it will not influence melanin production, and the migration of Fox03a to the nucleus results in a decrease in melanin production. In other words, it can be seen that the activation of Fox03a is an important factor capable of effectively inhibiting melanin production in skin cells.

### Test Example 2: Experiment on Decrease in Melanin Production by Treatment with Whitening Substance

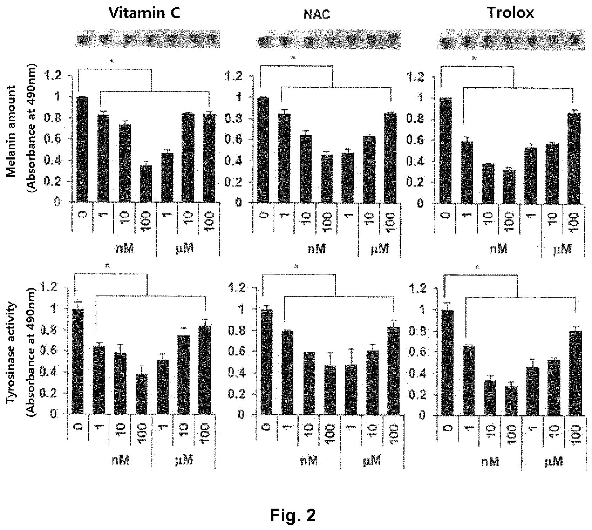

1×10⁶ MNT-1 cells (human melanoma cell line, Lonza, SWISS) were treated with 0, 1 nM, 10 nM, 100 nM, 1 µM, 10 µM and 100 µM of each of vitamin C, N-acetylcysteine (NAC) and Trolox, known as whitening substances, and were then cultured at 37°C for 48 hours. The cultured cells were lysed with 500 µL of LIPA buffer, and 500 µL of each of the cell lysates was centrifuged (Centrifuge 5415R, Eppendorf, Germany). The resulting pellets were separated, and the color thereof was observed. The content in each of the pellets was lysed with 200 µL of 1N sodium hydroxide. The absorbance of the lysate at 490 nm, which is specific for the melanin pigment, was measured, and the amount of the total protein was measured using a BCA protein assay kit (Pierce). The measurements were substituted into (absorbance) / (amount of total protein), and the amount of melanin was expressed as melanin / µg protein. The amount of melanin in the group treated with each of the whitening substances was calculated relative to 1 for the untreated group (0 nM), and the results of the calculation are shown in FIG. 2.

### Test Example 3: Evaluation of Inhibition of Tyrosinase Activity by Treatment with Whitening Substance

100 µL of 10 mM DOPA (dihydroxyphenylalanine) was added to 100 µL of each of the cell lysates prepared in Test Example 2, and then the lysates were incubated at 37°C for 1 hour, after which the absorbance of the lysates at 490 nm was measured to determine the amount of dopachrome produced by the action of tyrosinase. The absorbance (OD₄₉₀) of each test group was calculated relative to 1 for the untreated group (0 nM) to determine the ability of each test substance to inhibit tyrosinase activity, and the results of the calculation are shown in FIG. 2.

### Test Example 4: Observation of Migration of FoxO3a to Nucleus by Treatment with Whitening Substance

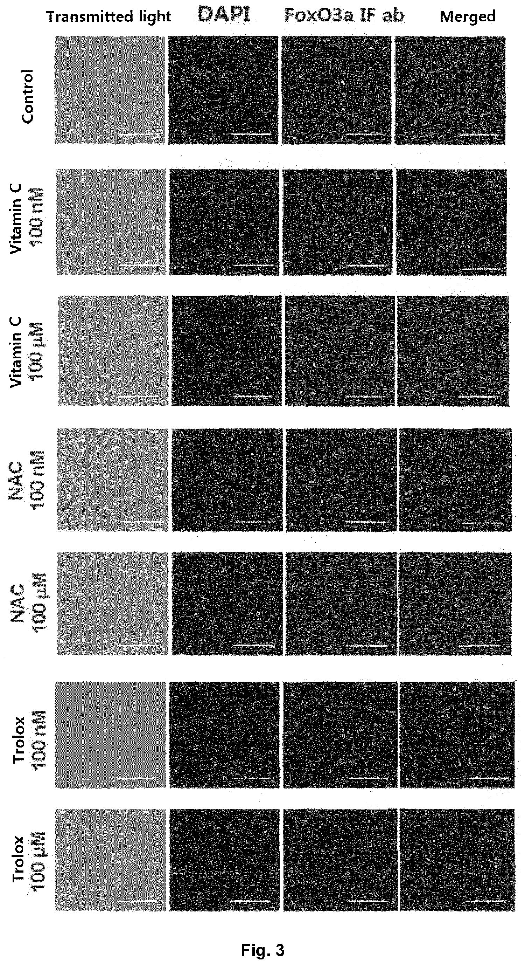

1×10⁶ MNT-1 cells (human melanoma cell line, Lonza, SWISS) were treated with 100 nM and 100 µM of each of vitamin C, N-acetylcysteine (NAC) and Trolox, known as whitening substances, and were then cultured at 37°C for 48 hours. As a control, untreated cells were used. The cultured cells were stained with each of DAPI (nuclear staining dye-blue) and Fox03a IF antibody (red) (Cell Signaling Technology) and observed with an EVOS FL digital fluorescence microscope (Advanced Microscopy Group), and the results of the observation are shown in FIG. 3.

From the results in FIG. 3, it can be seen that, when the cells were treated with each of vitamin C, N-acetylcysteine (NAC) and Trolox, the migration of Fox03a to the nucleus was observed. In addition, it can be seen that the migration of Fox03a to the nucleus was not observed at a concentration of 100 µM in each of the test groups, but was clearly observed at a concentration of 100 nM.

From the results of Test Examples 2 to 4, it can be seen that the substance, which exhibits a whitening effect at the concentration at which the migration of FoxO3a to the nucleus was observed and which mediates the activation of FoxO3a, is an anti-aging/whitening substance that exhibits all anti-aging and antioxidant effects and a whitening effect. Thus, it can be seen that all the anti-aging and antioxidant effects and whitening effect of a test substance can be simultaneously screened in a subjective and accurate manner by the screening method of the present invention.

### CITED REFERENCES

[1] KR 2010 0039077 A
[2] KR 2009 0054505 A
[3] KR 2010 0138918 A
[4] JP 2010 081913 A
[5] KR 2011 0057147 A
[6] WO 2012/057567
[7] article of J. M. Gillbro et al., "The melanogenesis and mechanisms of skin-lightening agents - existing and new approaches", International Journal Of Cosmetic Science, 1 January 2011, p.210-221.

## Claims

1. An *in vitro* method for screening a substance having a whitening effect, the method comprising analyzing whether or not a test substance activates Fox03a (Forkhead box 03A) or stimulates expression of Fox03a.

2. The method of claim 1, wherein the analyzing comprises the steps of:
a) treating skin cells with the test substance; and
b) observing migration of Fox03a in the skin cells treated with the test substance in step a).

3. The method of claim 2, wherein the analyzing further comprises step c) of determining that the test substance is the substance having the whitening effect, if the Fox03a migrates to a nucleus in the skin cells.

4. The method of claim 2 or 3, wherein the cells are keratinocytes, fibroblasts or melanocytes.

5. The method of claim 2, wherein in a) the skin cells are treated with a test substance at a concentration from 1 nM to 100nM.

6. The method of claim 1, wherein the activation or stimulation of expression of Fox03a is analyzed using Western blotting, ELISA or RT-PCR.

7. The method of claim 6, wherein the test substance is determined to be the substance having the whitening effect, if the test substance stimulates the expression of Fox03a.

8. The method of claim 6 or 7, wherein the test substance has both a whitening effect and an anti-aging effect.

## Patentansprüche

1. *In-vitro*-Verfahren zum Screenen einer Substanz mit Aufbleicheffekt, wobei das Verfahren umfasst das Analysieren, ob eine Testsubstanz Fox03a (Forkhead box 03A) aktiviert oder die Expression von Fox03a stimuliert, oder nicht.

2. Verfahren nach Anspruch 1, worin das Analysieren die Schritte umfasst:
a) Behandeln von Hautzellen mit der Testsubstanz; und
b) Beobachten der Migration von Fox03a in den mit der Testsubstanz wie in Schritt a) behandelten Hautzellen.

3. Verfahren nach Anspruch 2, worin das Analysieren ferner umfasst einen Schritt c), mit dem festgestellt wird, dass die Testsubstanz die Substanz mit Bleicheffekt ist, wenn Fox03a in einen Kern der Hautzellen wandert.

4. Verfahren nach Anspruch 2 oder 3, worin die Zellen Keratinozyten, Fibroblasten oder Melanozyten sind.

5. Verfahren nach Anspruch 2, worin in a) die Hautzellen mit der Testsubstanz bei einer Konzentration von 1 nM bis 100 nM behandelt werden.

6. Verfahren nach Anspruch 1, worin die Aktivierung oder Stimulation der Expression von Fox03a unter Verwendung von Western Blotting, ELISA oder RT-PCR analysiert wird.

7. Verfahren nach Anspruch 6, worin die Testsubstanz als Substanz mit Bleicheffekt festgestellt wird, wenn die Testsubstanz die Expression von Fox03a stimuliert.

8. Verfahren nach Anspruch 6 oder 7, worin die Testsubstanz sowohl einen Bleicheffekt als auch einen Anti-Aging-Effekt aufweist.

## Revendications

1. Procédé *in vitro* de criblage d'une substance ayant un effet blanchissant, le procédé comprenant le fait d'analyser si oui ou non une substance d'essai active Fox03a (protéine Forkhead box 03A) ou stimule l'expression de Fox03a.

2. Procédé selon la revendication 1, dans lequel l'analyse comprend les étapes de :
a) traitement de cellules de la peau avec la substance d'essai ; et
b) observation de la migration de Fox03a dans les cellules de la peau traitées avec la substance d'essai à l'étape a).

3. Procédé selon la revendication 2, dans lequel l'analyse comprend en outre l'étape c) de détermination que la substance d'essai est la substance ayant l'effet blanchissant, si la Fox03a migre vers un noyau dans les cellules de la peau.

4. Procédé selon la revendication 2 ou 3, dans lequel les cellules sont des kératinocytes, des fibroblastes ou des mélanocytes.

5. Procédé selon la revendication 2, dans lequel en a) les cellules de la peau sont traitées avec une substance d'essai à une concentration de 1 nM à 100 nM.

6. Procédé selon la revendication 1, dans lequel l'activation ou la stimulation de l'expression de Fox03a est analysée par Western blot, ELISA ou RT-PCR.

7. Procédé selon la revendication 6, dans lequel la substance d'essai est déterminée comme étant la substance ayant l'effet blanchissant, si la substance d'essai stimule l'expression de Fox03a.

8. Procédé selon la revendication 6 ou 7, dans lequel la substance d'essai a à la fois un effet blanchissant et un effet antivieillissement.
